(19) Europäisches Patentamt
European Patent Office
Office européen des brevets

(11) **EP 1 165 473 B1**

(12) **EUROPEAN PATENT SPECIFICATION**

(45) Date of publication and mention
of the grant of the patent:
**23.06.2004 Bulletin 2004/26**

(21) Application number: **99913593.2**

(22) Date of filing: **08.04.1999**

(51) Int Cl.⁷: **C07C 15/24**, C07C 4/18,
C07C 5/27, B01J 23/883,
B01J 23/882, C07C 7/13,
C10G 45/00

(86) International application number:
**PCT/JP1999/001880**

(87) International publication number:
**WO 2000/061529 (19.10.2000 Gazette 2000/42)**

(54) **PROCESS FOR PREPARING 2,6-DIALKYLNAPHTALENE**

VERFAHREN ZUR HERSTELLUNG VON 2,6-DIALKYLNAPHTHALEN

PROCEDE DE PREPARATION DE 2,6-DIALKYLNAPHTALENE

(84) Designated Contracting States:
**BE CH DE FR GB IT LI NL SE**

(43) Date of publication of application:
**02.01.2002 Bulletin 2002/01**

(73) Proprietors:
• **KABUSHIKI KAISHA KOBE SEIKO SHO**
**Kobe-Shi, Hyogo-ken 651-0072 (JP)**
• **EXXONMOBIL OIL CORPORATION**
**Fairfax, VA 22037 (US)**

(72) Inventors:
• **MOTOYUKI, Masahiro,**
**Osaka Branch in Kobe Steel Ltd**
**Osaka-shi, Osaka-fu 541-0051 (JP)**
• **YAMAMOTO, Koji**
**Kobe-shi, Hyogo-ken 651-2271 (JP)**

• **SAPRE, Vishwanath, Ajit**
**Paulsboro, NJ 08066-0480 (US)**
• **McWILLIAMS, Paul, John**
**Paulsboro, NJ 08066-0480 (US)**
• **DONNELLY, Patricia, Susan**
**Paulsboro, NJ 08066-0480 (US)**

(74) Representative: **Kyle, Diana**
**Elkington and Fife LLP,**
**Prospect House,**
**8 Pembroke Road**
**Sevenoaks, Kent TN13 1XR (GB)**

(56) References cited:
EP-A- 0 889 016          WO-A-99/19278
US-A- 5 744 670

**Description**

BACKGROUND OF THE INVENTION

Field of the Invention:

The present invention relates to a process for producing 2,6-dialkylnaphthalene (DAN) and, in particular, 2,6-dimethylnaphthylene (2,6-DMN) from a mixture which contains alkylnaphthalene or naphthalene.

Discussion of the Background:

In the manufacture of high performance polyester resins such as polyethylene naphthalate polymer (PEN) or polybutyrene naphthalate polymer (PBN), 2,6-DMN is used as a precursor of 2,6-naphthalene dicarboxylic acid. This is because 2,6-DMN is easily oxidized to 2,6-naphthalene dicarboxylic acid, when compared to other precursors such as 2,6-diisopropylnaphthalene or 2-methyl-6-isobutyrylnaphthalenes. There are many applications for PEN, e.g., films and bottles, such as long time recording type video film, Advanced Photo System, hot fill containers, refillable bottles and tire codes. PEN has good physical properties in strength, thermal resistance and gas barrier properties. Typical PBN applications include electronics, insulators and car parts. PEN and PBN have heretofore been too expensive, however, to effectively expand their markets due to the limited commercially viable processes for producing 2,6-DMN.

There have been many proposals for preparing 2,6-DMN. U.S. Pat. No. 4,795,847 (Weitkamp et al.) describes a process for the preparation of 2,6-dialkylnaphthalene by alkylating naphthalene or 2-alkyl-naphthalene with an alkylating agent in the presence of a zeolite (specially ZSM-5) as a catalyst.

U.S. Pat. No. 5,001,295 (Angevine et al) describes a process for preparing DMN by using 2-monomethylnaphthalene and naphthalene as a feedstock and a synthetic zeolite (MCM-22) as a catalyst, and it shows MCM-22 catalyst is more effective than ZSM-5 in alkylation of 2-NDAN and naphthalene.

However, the above methods provide only unit operation (i.e batch) for alkylation of 2-MMN, which is an expensive feedstock and is not commercially available in a large amounts.

U.S. Pat. No. 4,990,717 (Sikkenga) and 5,073,670 (Sikkenga et al.) describe a multistep process to produce 2,6-DMN from o-xylene and butadiene, which consists of;

1) preparation of 5-(o-tolyl)-pentene-2(OTP) by alkenylation of o-xylene with butadiene in the presence of a catalyst such as an alkali metal catalyst,
2) preparation of 1,5-dimethyltetralin (1,5-DMT) by cyclization of OTP in the presence of a catalyst such as platinum and copper on an ultra stable zeolite catalyst;
3) preparation of 1,5-dimethylnaphthalene (1,5-DMN) by dyhydrogenation of 1,5-DMT in the presence of a catalyst such as platinum and rhenium and gamma alumina; and
4) preparation of DMN mixture which is rich in the desirable 2,6-DMN, 1,6-DMN and 1,5-DMN by isomerization of 1,5-DMN in the presence of a catalyst such as a beta-zeolite catalyst.

If a process for separating 2,6-DMN from a DMN mixture were combined with the above steps, a complete process to produce purified 2,6-DMN could be provided.

As multiple steps complicate a process plant and increase the cost, it is not clear that the conventional processes could provide a process suitable for an economical preparation of purified 2,6-DMN.

In addition, it is very difficult to separate 2,6-DMN from other isomers by conventional separation methods such as distillation and cooling crystallization because;

1) There are very small differences in the boiling points of DMN isomers, and, in particular, between 2,6-DMN and 2,7-DMN wherein the difference in boiling points is only 0,3°C, and it is nearly impossible to separate 2,6-DMN by distillation.
2) The cooling of DMN isomer mixture solution of 2,6-DMN purification forms a precipitate of very fine 2,6-DMN crystals in suspension, and thus separation of the 2,6-DMN is extremely difficult.

Koide et al U.S. 4,992,619 reports a method for separating a methyl derivative of naphthalene from a mixture of materials in high purity by crystallization under pressure.

Moritoki et al U.S. 4,784,766 reports a pressure crystallization apparatus.

Accordingly, new and more efficient methods for commercially preparing dialkylnaphthalenes are sought.

EP 889016 is an invention related to a process to produce 2,6-dialkylnaphthalene (DAN) from naphthalene (NL) as raw material. The availability of NL is rather limited and NL is expensive. On the other hand, this invention is related to a process to produce 2,6-DAN from alkylnaphthalenes as a mixture of NL, MMN (mono methyl naphthalene), DMN and PAN (polyalkylnaphthalenes), etc., which are much more available and which price is much lower. Typically, such alkylnaphthalene stream is available in low value refinery streams (Typical example is light cycle oil produced from FFC) or ethylene cracker bottoms. Their typical price is similar to fuel value.

W099/19278, which is prior art under Articles 54(3) and (4) EPC, discloses a process of preparing a 2,6-dialkylnaphthalene comprising steps of alkylating monoalkylnaphthalene with an alkylating agent in an alkylation zone and transalkylating naphthalene with dialkylnaphthalene other than 2,6-dialkylnapthalene in a transalkylation zone.

SUMMARY OF THE INVENTION

According to one object of the invention, a method of preparing 2,6-dialkylnaphthalene is provided.

According to another object of the invention, a method of preparing 2,6-dimethylnaphthalene is provided.

These and other objects of the present invention are made possible by a process for producing 2,6-dialkylnaphthalene from a feedstock including hydrocarbons which contains at least one component selected from the group consisting of dialkylnaphthalene isomers, monoalkylnaphthalene isomers, polyalkylnaphthalenes, and naphthalene including the following steps:

I. separating a product fed from step III and, optionally, the feedstock into a fraction containing naphthalene, a fraction containing monoalkylnaphthalene, a fraction containing dialkylnaphthalene and a fraction containing remaining products;
II. separating and purifying 2,6-dialkylnaphthalene from the dialkylnaphthalene fraction of step I;
III. dealkylating the feedstock and the fraction containing the remaining products of step I and feeding the product of dealkylation to step I;
IV. alkylating the fractions containing naphthalene and monoalkylnaphthalene of step I,

wherein the hydrocarbon feedstock is fed to step III and, optionally, step I.

BRIEF DESCRIPTION OF THE DRAWINGS

Fig. 1 shows the preferred scheme of a process in accordance with Claim 1.
Fig. 2 shows the preferred scheme of a process in accordance with Claim 2.
Fig. 3 shows the preferred scheme of a process in accordance with Claim 3.
Fig. 4 shows the preferred scheme of a process in accordance with Claim 4.
Fig. 5 shows the preferred scheme of a process in accordance with Claim 5.
Fig. 6 shows the preferred scheme of a process in accordance with Claim 6.

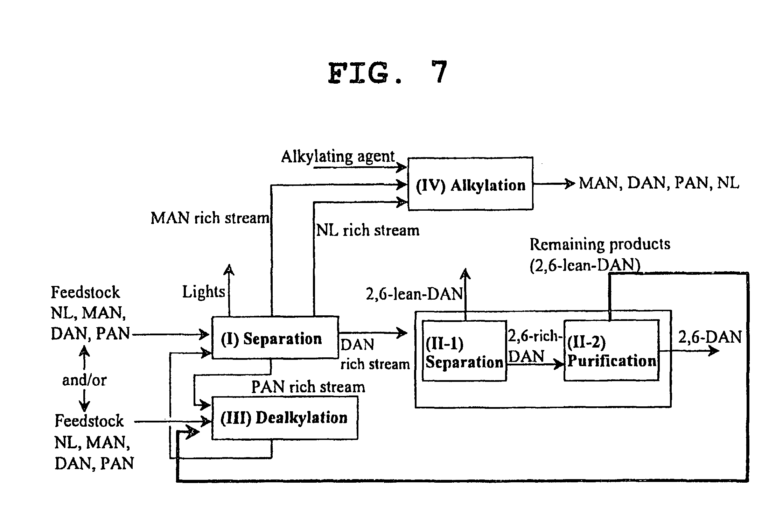

Fig. 7 shows the preferred scheme of a process in accordance with Claim 8.
Fig. 8 shows the preferred scheme of a process in accordance with Claim 9.
Fig. 9 shows the results of Example 7.
Fig. 10 shows the results of Example 12.
Fig. 11 shows the results of Example 13.

DESCRIPTION OF THE PREFERRED EMBODIMENTS

A more complete appreciation of the invention and many of the attendant advantages thereof will be readily obtained as the same becomes better understood by reference to the following detailed description when considered in connection with the accompanying drawings, which is not intended to be limiting unless otherwise specified.

Preferably, the present invention may be applied to any feedstock of hydrocarbons that contains alkylnaphthalenes such as naphthalene, MMN (monomethylnaphthalene) and DMN isomers. The present invention provides an effective production process of 2,6-dialkylnaphthalene (DAN), in particular, 2,6-dimethylnaphthalene (DMN) as high-value added product by utilizing a non-valuable feedstock.

In particular, LCO (Light Cycle Oil) and/or its heart cut fraction from FCC (Fluid Catalyst Cracking) or HC (Hydrocracker) is a preferable example of a feedstock. Although such feedstock usually contains alkylnaphthalenes at about 20 to 45 weight % of the feed stream, it has the following problems and difficulties for further processing:

1) Co-boilers

For example, LCO usually contains many components such as light paraffins and mono-aromatics with long chain alkyl-groups, which have similar boiling points to naphthalene, MMN and DMN (Co-boilers). It is very hard to separate alkylnaphthalenes from their co-boilers by distillation alone for further processing such as an alkylation step.
Therefore, if untreated, the co-boilers reduce the efficiency of the reactors. In the worst case, the co-boilers accumulate in the recycling streams in the process.

2) Polyalkylnaphthalenes (PAN)

PANs such as tri-methylnaphthalenes, isopropylnaphthalenes and tetra-methylnaphthalenes are usually contained in the feed stream at about 10 to 20 weight % of the feed stream, i.e., almost one third to one half of the alkylnaphthalene contents. PAN is also produced at other process steps such as alkylation and isomerization as by-products. In the conventional art, no effective process to utilize PANs for the production of 2,6-dialkylnaphthalene has been proposed.

3) Sulfur and Nitrogen compounds

The feed stream contains sulfur and nitrogen compounds, which might poison the catalyst for alkylation and isomerization, and these compounds should be excluded from the recycling streams and products.
The inventors have found that dealkylation (DA), especially hydrodealkylation (HDA) is (i) effective not only for cracking and/or reforming the co-boilers to lighter fractions, which results in much easier separation from naphthalenes, monoalkylnaphthalenes and dialkylnaphthalenes; but also, (ii) surprisingly effective for producing 2,6-dialkylnaphthalene especially 2,6-DMN by means of DA, especially HDA of polyalkylnaphthalenes (PAN) such as tri-methylnaphthalenes and heavier alkylnaphthalenes, which are contained in the feedstock and are also produced at alkylation step as useless by-products; and, (iii) effective for production of naphthalene and monoalkylnaphthalene which is suitable for alkylation feedstock.
The discovery results in more effective production of 2,6-DMN, not only at the alkylation step but also in the overall process because the invention provides an effective utilization of PAN.
Although a high feedstock conversion (e.g. Conversion of MMN) of around 50 to 60% provides a higher yield of 2,6-DMN, PAN production also increases, as shown in the TABLE 4 of the conventional processes, U.S. Pat. No. 5,744,670 (Motoyuki et al) (DMN and PAN content after alkylation is 35% and 23% respectively at MMN conversion with 58.28%.). This causes naphthalene-ring loss and lower process yields if PAN is not utilized again in the recycled stream. Therefore, in order to minimize the loss, MMN conversion is limited to around 30% or so, which decreases 2,6-DMN yield.
As the present invention can provide the effective utilization (an additional production of 2,6-dialkylnaphthalene from DA of PAN) of PAN derived from the feedstock, and preferably from the feedstock and alkylation step, it allows much higher feedstock conversion at the alkylation step, of around 50% and greater, which also results in higher production of 2,6-dialkylnaphthalene. Further, the inventors found that 2,6-lean-dialkylnaphthalene as the remaining products of the purification of 2,6-dialkylnaphthalene can be dealkylated and changed to equilibrium distributions of dialkylnaphthalene isomers or 2,6-rich dialkylnaphthalene by producing naphthalene and monoalkylnaphthalene simultaneously.
Although the reaction mechanism of DA of 2,6-dialkylnaphthalene is believed to be completely different, the results and product composition of DA are similar to transalkylation and isomerization. This means that DA can take over the performance of transalkylation shown in the conventional processes, e.g., U.S. Pat. No. 5,744,670 effectively.
The present invention provides an effective production process for 2,6-dialkylnaphthalene as a high-value added product by utilizing a non-valuable feedstock.
As a preferable feedstock for the present process, any hydrocarbon feedstream containing alkylnaphthalenes including at least one component selected from the group consisting of dialkylnaphthalene isomers, monoalkylnaphthalene isomers, polyalkylnaphthalenes, and naphthalene, such as Light Cycle Oil (LCO) derived from Catalytically cracking petroleum oil may be used. Alternatively, as raw material, hydrocarbon feedstream such as LCO is pre-processed, and afterwards, its product is preferably used as a feedstock for the present process. The pre-processing preferably includes distillation (e.g. heart cutting), concentration, hydrotreating (HDT) to reform sulfur and nitrogen compounds, which are usually contained in feedstreams and which might poison the catalyst, de-sulfurization, de-nitrogenation and de-watering.
In the distillation and concentration as the pre-processing, it is preferable to separate light components such as monoaromatic compounds and non-aromatic light paraffins and heavy compounds such as tri-aroma and heavier compounds from alkylnaphthalene components.

The preferable conditions of HDT include a temperature of about 200 to 1,000°C, and more preferably 200 to 500°C, and a pressure of 0 to 250 atm (25.3 MPa) and preferably 5 to 50 atm (0.5066 to 5.0663 MPa) a hydrogen circulation rate of from about 500 to 3000 scf/bbl (89.05 to 534.3 m$^3$/m$^3$). The reaction is preferably accomplished utilizing a feed space velocity of about 0.1 to 10.0 h$^{-1}$.

One preferred example of a suitable catalyst for HDT is an activated alumina supported catalyst bearing a Group VIII metal oxide and a Group VI-A metal oxide, preferably nickel and molybdenum respectively. The oxide may be preferably treated at 600-1200 F (315.6 - 648.9°C) in the presence of sulfur compounds.

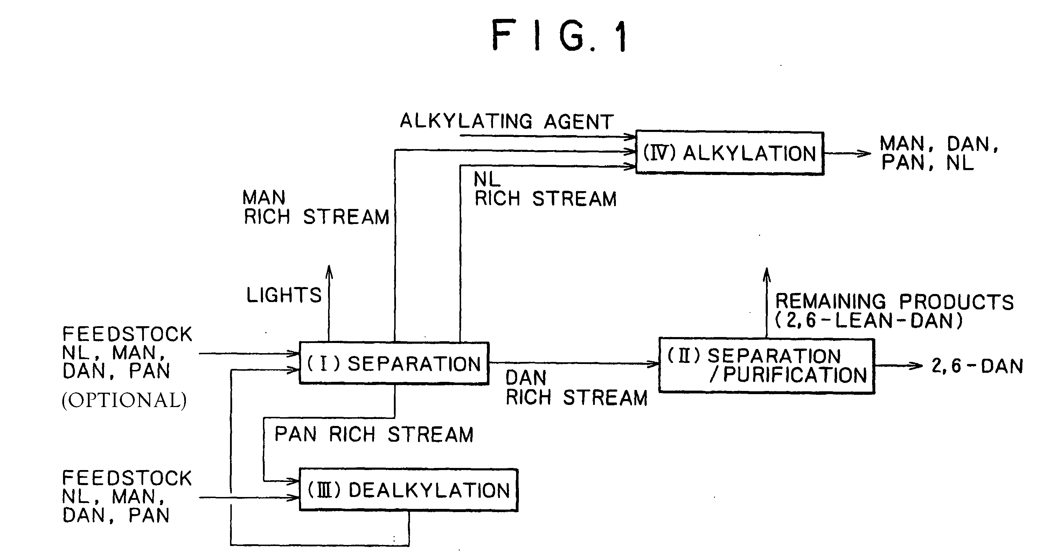

The feedstock can be fed to the separation of step I as well as to the dealkylation of step III. In the case that the feedstock is fed to step I, separating light components and heavy compounds from alkylnaphthalene components can be conducted in step I. On the other hand, in the case of the feedstock fed to step III, PAN components in the feedstock are reduced and changed to DMN or MMN components before separation of step I. In both cases effective production is obtained, since the remaining products of step I are fed to step III and the product of step III is fed to step I, as can be seen from Fig. 1.

For the separation of step 1, conventional techniques such as distillation may be used. In the case where the feed stream contains non-aromatic components having boiling points that are very similar to naphthalene and/or MMN, conventional solvent extraction techniques also can be applied in addition to the above mentioned distillation in step I.

The separation and purification of step II purifies the 2,6-DAN and separates the 2,6-lean-DAN from the DAN fraction of step I, as can be seen from Fig. 1.

Separation and purification of 2,6-dialkylnaphthalene of step II may be conducted by conventional methods known to those of ordinary skill in the art such as cooling crystallization and/or adsorption. For example, separation and purification may be affected by using a method of crystallization under high pressure. In general, a liquid mixture containing two or more substances is pressurized, and a certain substance in the mixture is solidified and separated from the residual liquid by the effect of the pressure. In other words, this method involves a separating and purifying technique wherein a liquid mixture containing two or more substances is placed in a tightly sealed pressure vessel, a portion of the desired substance, 2,6-dialkylnaphthalene, is solidified to form a solid-liquid co-existing state, the liquid is discharged from the co-existing system while maintaining the pressure of the solid-liquid co-existing system at a higher level than equilibrium pressure of the objective substance, then the solid remaining in the vessel is pressed for discharging the residual liquid between the solid particles and integrating the solid particles. This technique is generally described in U.S. 5,220,098.

The method involves injecting the slurry or liquid of the temperature of 70 to 120°C, preferably to 100°C, into a high pressure vessel for conducting a crystallization under high pressure, adiabatically pressurizing the vessel to a pressure of from 300 to 4000 kgf/cm$^2$ (29.42 to 392.3 MPa), preferably 500 to 2000 kgf/cm$^2$ (49.03 to 196.1 MPa) to increase the quantity, i.e. the amount of 2,6-dialkylnaphthalene crystals, whereby coexistence of solid-liquid phases exist at the high pressure conditions; discharging the liquid phase component from the high pressure vessel, the discharging being conducted under pressure, to increase the ratio of the solid phase relative to the liquid phase within the vessel; lowering the pressure of the residual liquid phase so as to dissolve partially and purify the product; discharging the residual liquid phase by applying pressure to the solid phase within the high pressure vessel whereby a 2,6-dialkylnaphthalene crystal block having a high purity is obtained within the high pressure vessel. By this technique, a purity of 2,6 dialkylnaphthalene (e.g. 2,6-dimethylnaphthylene) of 98 % by weight, preferably 99% by weight may be obtained.

In the separation and purification of step II, prior to the cooling crystallization and/or crystallization under high pressure, 2,6-dialkylnaphthalene can be pre-concentrated from dialkylnaphthalene mixture by fixed bed adsorptive separation system. As for the preconcentration of 2,6-DMN, it is preferable that the adsorption includes an adsorbent of a zeolite Y containing alkali metal and a desorbent of an organic solvent mainly composed of at least one component selected from the group consisting of hexane, octane, alkylbenzene, and cyclohexane. As alkylbenzene, mesitylene, o-xylene, and n-xylene are preferable.

At the cooling crystallization in the separation and purification step, since 2,6-DMN and 2,7-DMN form an eutectic crystal at the weight ratio of 0.7 (=2,6-DMN/2,7-DMN), only the low yield of 2,6-DMN is achieved. The theoretical 2,6-DMN separation yield is given by the following equations:

$$\text{Yield (\%)} = (1-0.7/k) \times 100,$$

where k=2,6-DMN/2,7-DMN at the feed of cooling crystallizer.

Therefore, it is most especially preferable to increase the ratio of 2,6-DMN/2,7-DMN for the higher yield of 2,6-DMN. The fixed bed adsorption can increase the ratio from 1.0 at the feed to 2.0 and more at output, which results in higher separation yields and much lower internal recycling amounts of the overall process.

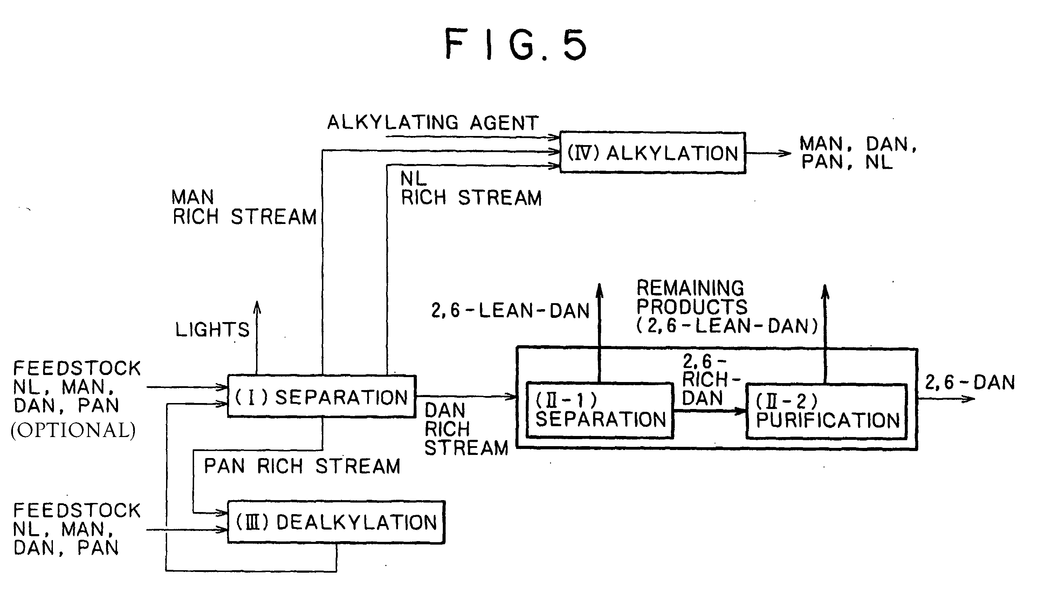

Preferably, for more effective production, the separation and purification of step II can be separated into separation section step II-1 and purification section step II-2, as can be seen from Fig. 5. In step II-1, the DAN fraction of step I is separated into 2,6-rich-DAN and 2,6-lean- DAN, and in step II-2, 2,6-DAN is purified from the 2,6-rich-DAN fraction from step II-1.

For example, separation of step II-1 may be preferably conducted by using distillation, and purification of step II-2 may be conducted by using cooling crystallization and/or crystallization under high pressure. By such a system, the 2,6-lean-DAN which contains little 2,7-DAN is separated by step II-1, and the 2,6-lean-DAN which contains much 2,7-DAN is separated by step II-2 as a remaining product of purification.

The conditions of HDA of step III include a temperature of about 200 to 1,000°C, and preferably 300 to 700°C, and a pressure of 0 to 250 atm (25.3 MPa) and preferably 5 to 150 atm (0.5066 to 15.1988 MPa) a hydrogen circulation rate of from about 500 - 3000 scf/bbl (89.05 to 534.3 m$^3$/m$^3$). The reaction is suitably accomplished utilizing a feed space velocity of about 0.1 to 10.0 hr$^{-1}$.

One example of a suitable catalyst for HDA, is an activated alumina supported catalyst bearing an oxide of Group VIII metal, preferably, chromium.

One more example of a suitable catalyst for HDA, is an activated alumina supported catalyst bearing an oxide of Group VIII metal and a Group VI metal, preferably, cobalt and molybdenum. The said oxide may be preferably pre-treated at a temperature of 600 - 1000 F (315.6 to 537.8°C) in the presence of organic sulfide.

Other preferred catalysts for HDA include catalysts including a metal selected from the group consisting of noble metal, nickel, and combinations thereof, and a synthetic zeolite characterized by an X-ray diffraction pattern including interplanar d-spacing and relative intensity $I/I_o$ x 100 as follows:

| | |
|---|---|
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS. |

The preferable noble metal is selected from the group consisting of platinum, palladium, and combinations thereof.

The conditions of alkylation of step IV preferably include a temperature of about 0 to 500°C, and preferably 240 and 450°C, and a pressure of between 0 to 250 atm (25.3 MPa) and preferably 1 to 50 atm (0.101 to 5.066 MPa). The mole ratio of alkylating agent to feed of monalkylnaphthylene or naphthalene can be from about 20:1 to 1:20, preferably from 10:1 to 1:10. The reaction is suitably accomplished utilizing a feed space velocity of about 0.1 to 10.0 hr$^{-1}$.

Preferred alkylating agents include alcohols, olefins, aldehydes, halides, and ethers. For example, methanol, dimethylether and polyalkylbenzene are preferred. Methanol and dimethylether are especially preferred.

A suitable catalyst for alkylation is a synthetic zeolite characterized by an X-ray diffraction pattern including interplanar d-spacing and relative intensity $I/I_o \times 100$ as follows:

| | |
|---|---|
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS. |

A suitable catalyst is MCM-22 (Exxon-Mobil Chemical Company) having above X-ray diffraction pattern.

Preferably, the alkylation can be carried out in any of the known reactors usually employed for alkylation. For example, a tubular reactor with a downflow of reactants over a fixed bed of catalyst can be employed.

In order to maintain high feedstock conversion, the injection of methanol to reactor can be performed, preferably, in multiple stages, and more preferably two stages. For example, one reactor with top and middle methanol feed, or two reactors in series with top and intermediate methanol feed are preferably used.

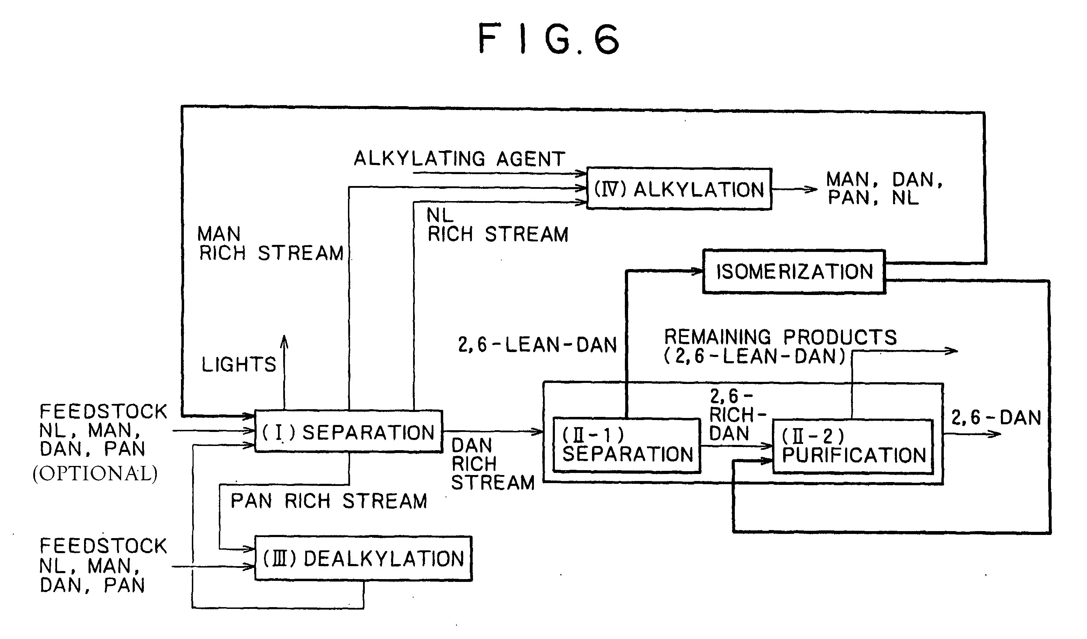

In a preferred embodiment, the 2,6-lean-DAN fraction from step II-1 may be subjected to isomerization conditions to provide for a dialkylnaphthalene fraction which has a greater content of 2,6-dialkylnaphthalene, as can be seen from Fig. 6. Preferably, the product of the isomerization may be fed to step I and/or step II-2 for more efficient recovery.

Preferred isomerization conditions are generally disclosed in co-pending application U.S. Application Serial No. 08/661,114, and are suitable for conducting simultaneous transalkylation of dialkylnaphthalene and naphthalene, and isomerization of dialkylnaphthalenes.

A preferred catalyst for isomerization is a synthetic zeolite characterized by an X-ray diffraction pattern including interplanar d-spacing and relative intensity $I/I_o$ x 100 as follows:

| | |
|---|---|
| $12.36 \pm 0.4$ | M-VS |
| $11.03 \pm 0.2$ | M-S |
| $8.83 \pm 0.14$ | M-VS |
| $6.18 \pm 0.12$ | M-VS |
| $6.00 \pm 0.10$ | W-M |
| $4.06 \pm 0.07$ | W-S |
| $3.91 \pm 0.07$ | M-VS |
| $3.42 \pm 0.06$ | VS. |

A suitable catalyst is MCM-22 (ExxonMobil Chemical Company) having above X-ray diffraction pattern.

Preferably, isomerization is conducted at a weight hourly space velocity (WHSV) of dialkylnaphthalenes of 0.1 to 10, preferably 0.5 to 5 $h^{-1}$, more preferably 0.75 to 1.5 $h^{-1}$.

Preferably, isomerization is conducted at a temperature of from 100 to 500°C, preferably 150 to 350°C, more preferably 200 to 300°C.

Preferably, isomerization is conducted at a pressure of atmospheric to 100 kgf/cm$^2$ (9.807 MPa), atmospheric to preferably 30 kcf/cm$^2$ (2.942 MPa).

During isomerization it is optionally preferable to co-feed hydrogen in an amount of 0.1 to 10 mol-$H_2$/mol-hydrocarbons.

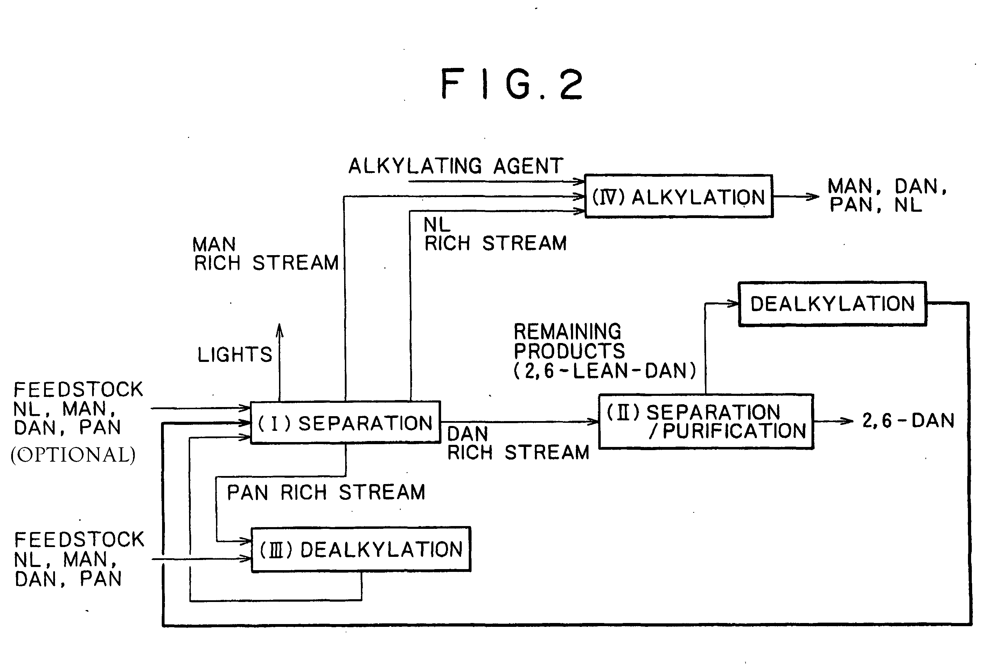

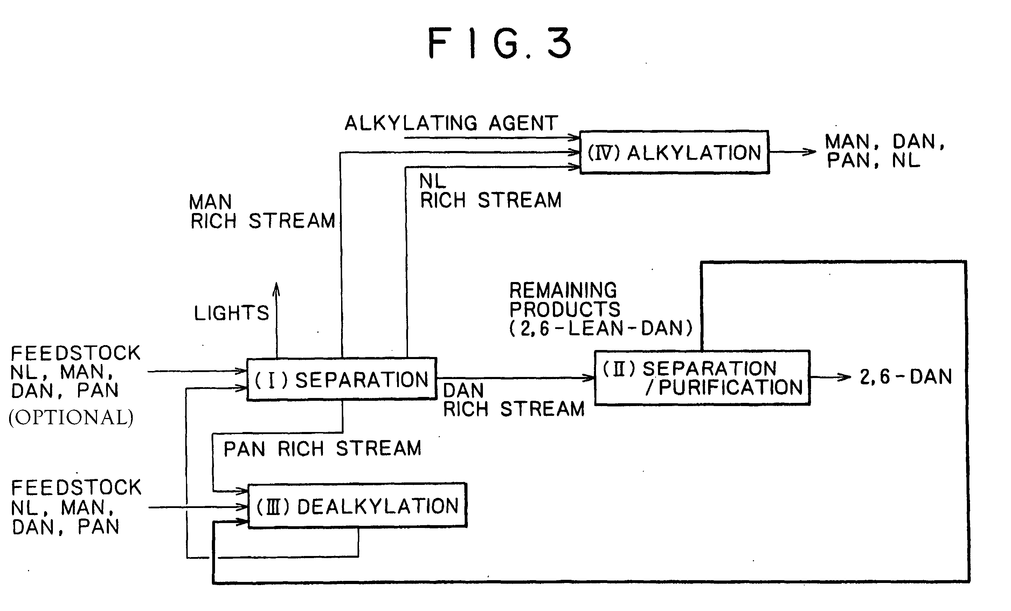

According to the preferred embodiment of Figures 2 or 3, 2,6-dialkylnaphthalene may be prepared from hydrocarbon feedstocks as follows:

I. separating a product fed from step III and, optionally, a feedstock into a fraction containing naphthalene, a fraction containing monoalkynaphthalene, a fraction containing dialkylnaphthalene and a fraction containing remaining products,

II. separating and purifying 2,6-dialkylnaphthalene from the dialkylnaphthalene fraction of step I,

IIIa. dealkylating a dialkylnaphthalene fraction after 2,6-dialkylnaphthalene is separated therefrom in step II and recycling a product of dealkylation to step I;

IIIb. dealkylating the feedstock and the fraction containing remaining products of step I and feeding the product of dealkylation to step I;

V. alkylating the fractions containing naphthalene and monoalkynaphthalene of step I.

In this process, 2,6-lean-DAN as remaining product of separation/purification of step II is dealkylated and fed to separation of step I. So, 2,6-DAN isomers in 2,6-lean-DAN may be changed to MMN or NL and can be alkylated in step IV.

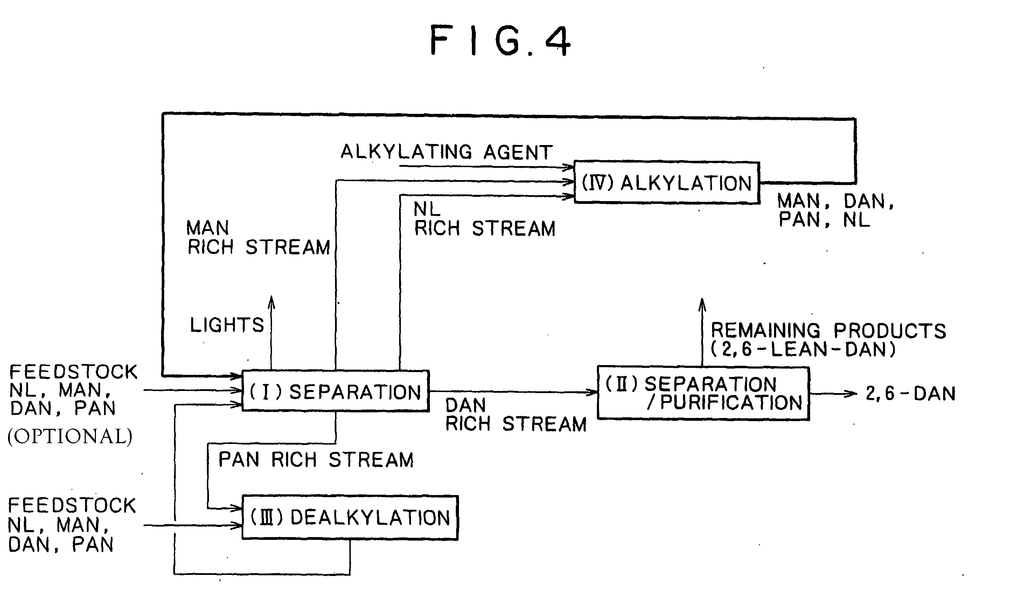

As for the preferred embodiment in Figure 4, the product of alkylation of step IV is fed to separation of step I. Accordingly, PAN produced in step IV can be separated in step I and fed to dealkylated in step III. Therefore, it enables to provide the effective utilization of PAN and allows much higher feedstock conversion at alkylation step, as already described.

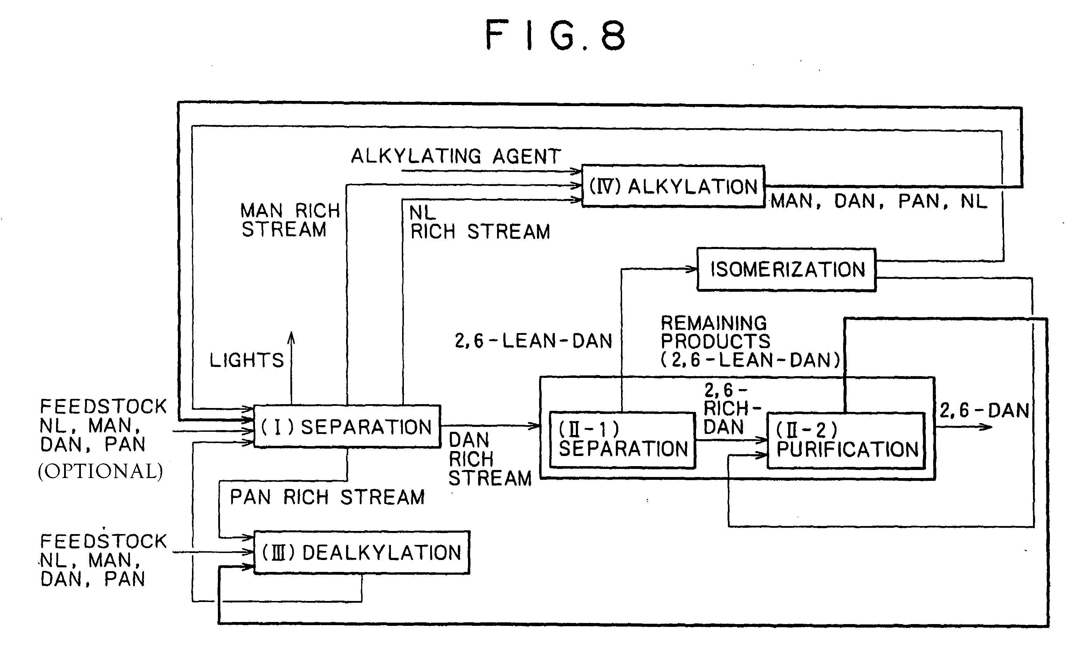

The process scheme of Figure 8 is a most preferable embodiment of the present invention.

EXAMPLES

Having generally described this invention, a further understanding can be obtained by reference to certain specific examples, which are provided herein for purposes of illustration only and are not intended to be limiting unless otherwise specified.

Example 1 Alkylation of MMN and Naphthalene:

A 153 g amount of MCM-22 catalyst is charged into a tubular reactor (volume:370 cm$^3$). As a feedstock for alkylation, 1-MMN, 2-MMN and naphthalene are used, and mixed at a molar ratio of 2.2 of 2-MMN/1-MMN, and a weight ratio of 3.0 of MMN's (1-MMN+2-MMN)/naphthalene.

Thereupon, the feedstock is supplied to the reactor (254 C, 5 kgf/cm$^2$ (0.490 MPa)) at a rate of 153.4 g/hr and 1.0 hr$^{-1}$ in WHSV with a feed of hydrogen at the rate of 1.8 ft$^3$/hr (0.051 m$^3$/hr). Four hours later, methanol, as an alkylating agent, is introduced into the reactor at 35.5 g/hr, and alkylation is conducted for 20 hours. The product obtained is analyzed by gas chromatography, and the results are summarized in Table 1.

TABLE 1

| (Alkylation of Monomethylnaphthalene and Naphthalene) | | |
|---|---|---|
| Component (wt%) | Before Reaction | After Reaction |
| dimethylnaphthalene | 0 | 17.19 |
| 2,6-DMN | 0 | 1.72 |
| 2,7-DMN | 0 | 1.20 |
| other isomers | 0 | 14.27 |
| monomethylnaphthalene | 73.63 | 60.10 |
| 2-MMN | 50.55 | 40.32 |
| 1-MMN | 23.08 | 19.78 |
| Naphthalene | 25.28 | 18.67 |
| other component | 1.00 | 3.91 |
| Evaluation | Before Reaction | After Reaction |
| NL conversion (%) | -- | 26.15 |
| 2-MMN/1-MMN | 2.2 | 2.04 |
| MMN conversion (%) | -- | 18.37 |
| 2,6-DMN/total DMN (%) | -- | 10.02 |
| 2,6-DMN/2,7-DMN | -- | 1.44 |

As can be seen from Table 1, the ratio of 2,6-DMN/2,7-DMN is over 1.1 and the ratio of 2-MMN/1-MMN is over 2.0.

Example 2 (alkylation)

153 g of MCM-22 were charged in the tubular reactor (volume: 370 cm$^3$). As a feedstock for alkylation, 1-MMN (purity 95.5%) and 2-MMN (purity 96.6%) were used, and mixed at the molar ratio of 2.2 of 2-MMN/1-MMN. Feedstock was supplied in the reactor (350°C) at the rate of 76.7 g/hr and 0.5 hr$^{-1}$ in WHSV for 4 hours. Thereafter, methanol was started to be supplied in the reactor at the rate of 17.3 g/hr and the reaction was proceeded for 20 hours. The obtained product was analyzed by gas chromatography, and the result is summarized in Table 2.

Table 2

| (Alkylation) | | |
|---|---|---|
| component (wt %) | before reaction | after reaction |
| * dimethylnaphthalene | 0 | 35.45 |
| 2,6-DMN | 0 | 5.12 |
| 2,7-DMN | 0 | 4.44 |
| other isomers | 0 | 25.89 |
| * monomethylnaphthalene | 98.66 | 41.16 |
| 2-MMN | 67.61 | 28.84 |
| 1-MMN | 31.05 | 12.32 |

Table 2   (continued)

| (Alkylation) | | |
|---|---|---|
| component (wt %) | before reaction | after reaction |
| * naphthalene | 0 | 0.19 |
| * other component (mainly PAN) | 1.53 | 23.20 |
| Evaluation | before reaction | after reaction |
| 2-MMN/1-MMN | 2.2 | 2.3 |
| MMN conversion (%) | - | 58.28 |
| 2,6-DMN/total DMN (%) | - | 14.45 |
| 2,6-DMN/2,7-DMN | - | 1.16 |

As can be seen from Table 2, the ratio of 2,6-DMN/2,7-DMN is over 1.1 and the ratio of 2-MMN/1-MMN is over 2.0.

Example 3 (Alkylation and Distillation)

Alkylation of MMN and naphthalene has been carried out for several months in the same manner described in Example 1 and about 400 kg of the product is collected. Distillation of the product is carried out by using a batch type distillation tower with a packed column. A number of theoretical trays of the tower is expected to be at least 50. The operation pressure at the top of the column is controlled between 15 and 36 Torr (2 KPa and 4.8 KPa) and distillation proceeds at a reflux ratio of 50 to 75.
The product is separated into 17 fractions by differences in boiling points as shown in Table 3.

Table 3

| (Alkylation and Distillation) | | | |
|---|---|---|---|
| | Amount (kg) | DMN concentration (%) | 2,6-DMN concentration (%) |
| Fraction-1-10 | 270.8 | 0.0 | not analyzed |
| Fraction-11 | 30.9 | 0.5 | not analyzed |
| Fraction-12 | 8.8 | 38.9 | not analyzed |
| Fraction-13 | 11.0 | 64.8 | 11.2 |
| Fraction-14 | 6.3 | 92.3 | 25.4 |
| Fraction-15 | 15.7 | 99.6 | 4.3 |
| Fraction-16 | 4.8 | 98.7 | 0.0 |
| Fraction-17 | 5.3 | 41.6 | 0.0 |
| Residue | 21.2 | 0.0 | 0.0 |

Example 4 (Hydrodealkylation)

A part of Fraction-17 and Residue shown in Table 3 are mixed to prepare the feedstock(Blend-A) for hydrodealkylation. A 50 g amount of $Cr_2O_3/Al_2O_3$ type catalyst produced by Sud-Chemie AG is charged into a tubular reactor. The reactor is heated gradually from ambient temperature to 662F (350 °C) to dry the catalyst while supplying hydrogen gas. Thereupon Blend-A is fed to the reactor at the rate of 50 g/hr and 1.0 hr$^{-1}$ in WHSV, while supplying hydrogen gas at 1.25 cf/hr (0.034 m$^3$/hr). Hydrodealkylation is carried out at 887F (475 °C) and 854 psig (5.99 MPa). The product is analyzed by GC and the results of hydrodealkylation are summarized in Table 4 below.
As shown in Table 4, $Cr_2O_3/Al_2O_3$ type catalyst is effective to enrich 2,6-DMN from 2,6-DMN lean feed.

Table 4

| (Hydrodealkylation) | | |
|---|---|---|
| | Feed (Blend-A) | HDA Product |
| Naphthalene | 0.0 | 3.11 |
| 2-MN | 0.0 | 15.68 |
| 1-MN | 0.0 | 3.71 |
| 2-EN | 0.0 | 0.57 |
| 1-EN | 0.0 | 0.26 |
| 2,6-DMN | 0.0 | 4.86 |
| 2,7-DMN | 0.0 | 4.58 |
| 1,3- + 1,7-DMN | 0.0 | 7.58 |
| 1,6-DMN | 0.0 | 2.92 |
| 2,3- + 1,4-DMN | 0.14 | 4.84 |
| 1,5-DMN | 0.0 | 0.39 |
| 1,2-DMN | 20.18 | 9.42 |
| 1,8-DMN | 0.0 | 0.12 |
| Unknowns before first DMN | 0.0 | 2.50 |
| Unknowns between DMN | 1.34 | 0.39 |
| Heavies Including Polymethylnaphthalenes | 78.34 | 39.08 |
| Total DMNs (%) | 20.28 | 34.71 |
| 2,6-DMN/Total DMNs (%) | 0.0 | 13.20 |

Example 5 Isomerization:

A 25 g amount of MCM-22 catalyst is charged into the tubular reactor (volume: 200 cm$^3$. The reactor is heated gradually from ambient temperature to 400°C to dry the catalyst while supplying nitrogen gas, and the flow of nitrogen gas is ceased when the temperature becomes stable at 400°C. Thereupon, 2.6-lean-DMN is supplied to the reactor at the rate of 25 g/hr and 1.0 hr$^{-1}$ in WHSV, and isomerization of DMN is carried out for four hours. The contents of the obtained product are analyzed by gas chromatography, and the results are summarized in Table 5.

TABLE 5

| (Isomerization) | | |
|---|---|---|
| Component (wt%) | before reaction | After reaction |
| dimethylnaphthalene | 98.09 | 80.10 |
| 2,6-DMN | 6.21 | 13.96 |
| 2,7-DMN | 8.48 | 8.66 |
| other isomers | 83.40 | 57.48 |
| monoethylnaphthalene | 0.20 | 9.77 |
| 2-MMN | 0.03 | 6.71 |
| 1-MMN | 0.17 | 3.06 |
| naphthalene | 0 | 0.78 |
| other component | 1.71 | 9.35 |
| evaluation | before reaction | after reaction |
| 2,6-DMN/total DMN (%) | 6.3 | 17.4 |

TABLE 5 (continued)

| (Isomerization) | | |
|---|---|---|
| evaluation | before reaction | after reaction |
| 2,6-DMN/2,7-DMN | 0.73 | 1.61 |

Example 6 Separation and Purification:

(1) Crystallization under High Pressure Crystallization

A 1,505 g amount of DMN isomers is supplied into the high pressure crystallizer (KOBELCO 1.5L type), and 236 g of 2,6-DNN crystals (purity 87%) are separated under the condition of 2000 kgf/cm$^2$ (196.1 MPa) and 45°C.

(2) Cooling Crystallization

Using a vessel for crystallization (3 liter), 2,001 g of DMN isomers is cooled quickly from 50 to 40°C with slow stirring. Then, 0.5 g of seed crystals are charged to the vessel which is kept at a temperature at 40°C for an hour. Thereupon, the feedstock is cooled to 10°C at 2°C/min. A 360 g amount of 2,6-DMN crystals (purity 68 %) is separated by filtration under pressure.

The results of separation by both crystallization under high pressure and cooling crystallization are summarized in Table 6.

TABLE 6

| (Separation and Purification) | | | | |
|---|---|---|---|---|
| CRYSTALLIZATION UNDER HIGH PRESSURE | | | | |
| Component (g) | | before crystallization | crystal | filtrate |
| | 2,6-DMN | 301 | 205 | 96 |
| | 2,7-DMN | 232 | 22- | 210 |
| | other DMN | 972 | 9 | 963 |
| | TOTAL | 1505 | 236 | 1269 |
| 2,6-DMN/2,7-DMN | | 1.3 | -- | 0.5 |
| 2,6-DMN/total DMN | | 20.0% | -- | 7.6% |
| purity of crystal | | -- | 87% | -- |
| recovery of 2,6-DMN | | -- | 68% | -- |
| yield of 2,6-DMN | | -- | 13.6% | -- |
| COOLING CRYSTALLIZATION | | | | |
| Component (g) | | before crystallization | crystal | filtrate |
| | 2,6-DMN | 400 | 244 | 156 |
| | 2,7-DMN | 308 | 67 | 241 |
| | other DMN | 1293 | 49 | 1244 |
| | TOTAL | 2001 | 360 | 1641 |
| 2,6-DMN/2,7-DMN | | 1.3 | -- | 0.65 |
| 2,6-DMN/total DMN | | 20.0% | -- | 9.5% |
| purity of crystal | | -- | 68% | -- |
| recovery of 2,6-DMN | | - | 61% | -- |

TABLE 6   (continued)

| (Separation and Purification) | | | |
| --- | --- | --- | --- |
| COOLING CRYSTALLIZATION | | | |
| Component (g) | before crystallization | crystal | filtrate |
| yield of 2,6-DMN | -- | 12.2% | -- |
| "Recovery of 2,6-DMN" means the content of 2,6-DMN in the crystals against the content of 2,6-DMN in the feedstock.<br><br>"Yield of 2,6-DMN" means the content of 2,6-DMN in the crystal against the total weight of feedstock. | | | |

As shown in Table 6, the yield of 2,6-DMN by crystallization under high pressure is much higher than by cooling crystallization. Further, the 2,6-DMN/total-DMN ratio of the filtrate by crystallization under high pressure is less than 8%. Therefore, the filtrate is more effective as a feedstock for transalkylation and isomerization of 2,6-lean-DMN.

Furthermore, when an attempt is made to increase the purity of crystals by cooling crystallization, the yield of 2,6-DMN decreases drastically.

Example 7 (Purification)

Pre-condensation of 2,6-DMN from DMN mixture (Table 7) was tried by cooling crystallization and a 2,6-DMN rich cake, which is to be used as feedstock for the crystallization under high pressure, was separated by bench scale pressure filtration unit.

Purification of 2,6-DMN from the 2,6-DMN rich cake was carried out by the crystallation under high pressure method using Kobelco's HPC test machine.

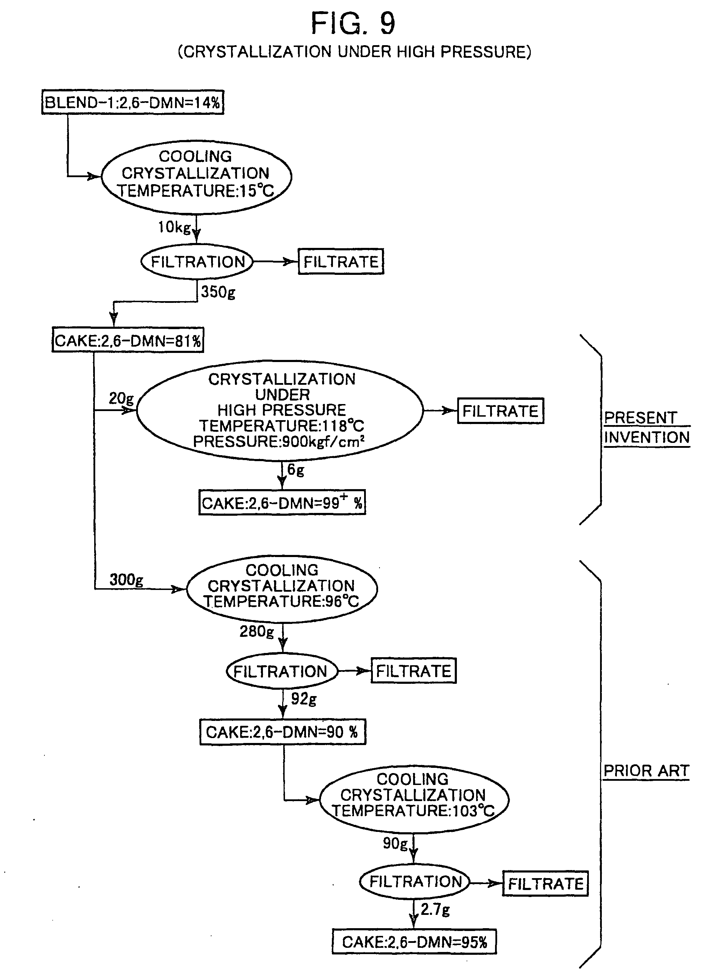

Several series of experiments were performed and results are summarized in Figure 9.

As can be seen in Figure 9, crystallization under high pressure achieves more effective purification performance in separation yield and 2,6-DMN purity by single stage crystallization than does two-stage Cooling Crystallization.

TABLE 7

| (Composition of DMN Mixture) | |
| --- | --- |
| | DMN mixture |
| Sulfur (ppm) | 430 |
| Nitrogen (ppm) | 140 |
| 2,6-DMN | 13.8 |
| 2,7-DMN | 14.1 |
| 1,6-DMN | 7.1 |
| 1,3- & 1,7-DMN | 20.5 |
| 1,4-DMN | - |
| 1,2- & 1,5-DMN | 0.4 |
| 2,3-DMN | 0.5 |
| Others | 43.6 |

Example 8 (Distillation)

Two types of batch distillation tower are used for the separation of alkylnaphthalenes from LCO. One of the distillation tower (Fractioneer-A) has 167 l (0.167 m$^3$) still and 32 ft (0.75 m) long column with PRO-PAK (Scientific Development Company) and the other distillation tower (Fractioneer-B) has 27 liters still with an 11 foot long column with PRO-PAK. 164 kg of LCO is charged into the Fractioneer-A and distillation is carried out at a reflux ratio of 50 and a pressure of 60 Torr (8.0 KPa). 80.5 liters are taken at a take off rate of 0.7 liters per hour.

Then 25 kg of the residue in the still of Fractioneer-A is taken out after the first distillation and charged into Fractioneer-B. Another batch distillation is carried out at a reflux ratio of 50 and a pressure of 50 Torr (6.67 Kpa). 14 liters are taken at a take off rate of 125 ml per hour. The components of the product (Blend-B) obtained from the above-mentioned two step distillation are shown in Table 8.

Example 9 (Hydrodealkylation)

A 70 g amount of $Cr_2O_3/Al_2O_3$ type catalyst produced by Sud-Chemie AG is charged into a tubular reactor. The reactor is heated gradually from ambient temperature to 932 F (500 °C) to dry the catalyst while supplying hydrogen gas. Thereupon distillation product (Blend-B) obtained from Example 8 is supplied to the reactor at the rate of 70 g/hr and 1.0 hr$^{-1}$ in WHSV, while supplying hydrogen gas at 0.98 scf/hr (0.028 m$^3$/hr). The hydrodealkylation reaction is carried out at 933 F (500.6 °C) and 1138 psig (7.95 MPa). The product is analyzed by GC and the results of hydrodealkylation are summarized in Table 8 below.

Example 10 (Hydrodealkylation)

A 70 g amount of $CoO/MoO_3/Al_2O_3$ type catalyst produced by Akzo Chemicals Inc. is charged into a tubular reactor. The reactor is heated gradually from ambient temperature to 300F (148.9 °C) with nitrogen flow at 5 scf/hr (0.142 m$^3$/hr). Then the flow gas is switched to hydrogen at 2 scf/hr (0.057 m$^3$/hr) and pressure is increased to 500 psig (3.55 MPa). Catalyst is contacted with an organic sulfide (Kerosene with 1.0% of Dimethyldisulfide) for sulfiding while supplying hydrogen gas and then temperature is raised to 650 F (343.3 °C). Thereupon distillation product (Blend-B) obtained from Example 8 is fed to the reactor at the rate of 70 g/hr and 1.0 hr$^{-1}$ in WHSV, while supplying hydrogen gas at 5scf/hr (0.028 m$^3$/hr). Hydrodealkylation is carried out at 932 F (500 °C) and 425 psig (9.93 Mpa) The product is analyzed by GC and the results of hydrodealkylation are summarized in Table 8 below.

As shown in Table 8, both $Cr_2O_3/Al_2O_3$, and $CoO/MoO_3/Al_2O_3$ type catalyst are effective to enrich DMN isomers from DMN lean feed.

Table 8

| (Hydrodealkylation) | | | |
|---|---|---|---|
| | Example 7 | Example 8 | Example 9 |
| | Distillation Product (Blend-B) | HDA Product | HDA Product |
| Catalyst | | $Cr_2O_3/Al_2O_3$ | $CoO/MoO_3/Al_2O_3$ |
| naphthalene | 0.0 | 1.33 | 4.75 |
| 2-MN | 0.0 | 4.31 | 9.48 |
| 1-MN | 0.0 | 1.05 | 2.44 |
| 2-EN | 0.0 | 1.56 | 2.13 |
| 1-EN | 0.0 | 0.36 | 0.44 |
| 2,6-DMN | 0.0 | 2.46 | 3.53 |
| 2,7-DMN | 0.0 | 2.56 | 3.76 |
| 1,3- + 1,7-DMN | 0.0 | 2.55 | 3.82 |
| 1,6-DMN | 0.0 | 1.43 | 2.17 |
| 2,3- + 1,4-DMN | 0.0 | 1.18 | 1.44 |
| 1,5-DMN | 0.0 | 0.24 | 0.34 |
| 1,2-DMN | 0.04 | 0.40 | 0.32 |
| 1,8-DMN | 0.04 | 0.25 | 0.47 |
| Unknowns before first DMN | 0.0 | 8.67 | 17.68 |
| Unknowns between DMN | 0.0 | 0.54 | 0.68 |
| Heavies including polymethylnaphthalenes | 99.92 | 71.11 | 46.56 |
| Total DMNs (%) | 0.08 | 11.07 | 15.85 |
| 2,6-DMN-Total DMNs | 0.0 | 22.22 | 22.27 |

Example 11 (Distillation and Hydrotreating)

164 kg of LCO is charged into the Fractioneer-A and distillation is carried out at a reflux ratio of 50 and a pressure of 60 Torr (8 KPa) 120 liters are taken at a take off rate of 0.7 liters per hour and prepared for hydrotreating feedstock as Blend-C. $NiO/MoO_3/Al_2O_3$ type catalyst produced by Akzo Chemicals Inc. is chosen as a hydrotreating catalyst and charged into a tubular reactor.

After drying and sulfiding catalyst, then Blend-C is fed into the reactor at the rate of 0.43 hr$^{-1}$ in WHSV and hydrotreating is carried out at 400 psig (2.86 MPa) and 726F (385.6 °C), while supplying hydrogen gas at 3495 scf/bbl at (622.5 $m^3/m^3$). The results of hydrotreating are summarized in Table 9 below.

As shown in Table 9, $NiO/MoO_3/Al_2O_3$ type catalyst is effective to reduce the nitrogen and/or sulfur compounds in LCO with minimum loss of DMN isomers.

Table 9

| (Distillation and Hydrotreating) | | | | | | | |
|---|---|---|---|---|---|---|---|
| | Nit. ppm | Sul. ppm | API | 400F+ Conv. (%) | 400F+ Conv. (%) | 500F+ Conv. (%) | Total DMNs wt% |
| Blend-C | 230 | 388 | 18.7 | - | - | - | 26.56 |
| HDT Product | 7 | 5 | 20.6 | 0.6 | 7 | 21 | 21.24 |

Example-12 (Adsorption)

2,6-DMN and 2,7-DMN are mixed and dissolved into iso-octane at 2.0 wt% of concentration respectively. Then DMN-isooctane solution is fed to the adsorption column (4.6 mm ID and 500 mmL) packed with K-Y zeolite at the rate of 0.50 ml/min, while column temperature is controlled at 158 F (70 °C) Time course data of the DMN concentration in the effluent is gathered by GC analysis and breakthrough curve in adsorption step is obtained.

After the adsorption step, liquid feed is switched to the pure iso-octane and effluent is also analyzed to gather the time course data of the DMN concentration in the effluent.

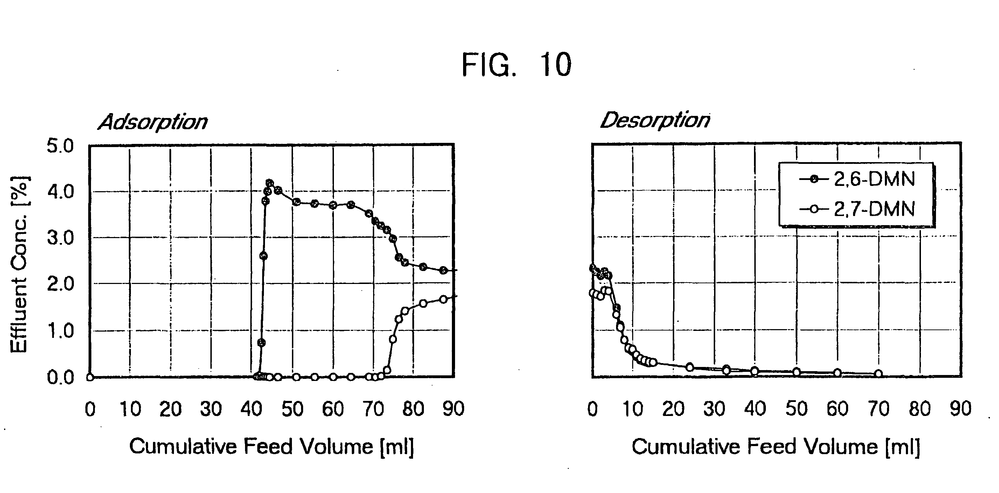

The results of the breakthrough curve and desorption curve are summarized in Fig. 10. As shown in the figure, 2,7-DMN is priorly adsorbed in zeolite compared to 2,6-DMN and it is obviously possible to improve 2,6-/2,7-DMN by contacting DMN isomers with the K-Y type zeolite.

Example-13

Another adsorption test is carried out in the same manner described in Example 12 catalyst produced by Akzo Chemicals Inc. is chosen as a hydrotreating catalyst and charged into a tubular reactor.

After drying and sulfiding catalyst, then Blend-C is fed into the reactor at the rate of 0.43 hr$^{-1}$ in WHSV and hydrotreating is carried out at 2.86 MPa and 385.6 °C, while supplying hydrogen gas at 622.5 $m^3/m^3$. The results of hydrotreating are summarized in Table 9 below.

As shown in Table 9, $NiO/MoO_3/Al_2O_3$ type catalyst is effective to reduce the nitrogen and/or sulfur compounds in LCO with minimum loss of DMN isomers.

Table 9

| (Distillation and Hydrotreating) | | | | | | | |
|---|---|---|---|---|---|---|---|
| | Nit. ppm | Sul. ppm. | API | 400F+ Conv. (%) | 400F+ Conv. (%) | 500F+ Conv. (%) | Total DMNs wt% |
| Blend-C | 230 | 388 | 18.7 | - | - | - | 26.56 |
| HDT Product | 7 | 5 | 20.6 | 0.6 | 7 | 21 | 21.24 |

Example-12 (Adsorption)

2,6-DMN and 2,7-DMN are mixed and dissolved into iso-octane at 2.0 wt% of concentration respectively. Then DMN-isooctane solution is fed to the adsorption column (4.6 mm ID and 500 mmL) packed with K-Y zeolite at the rate of 0.50 ml/min, while column temperature is controlled at 70 °C. Time course data of the DMN concentration in the effluent is gathered by GC analysis and breakthrough curve in adsorption step is obtained.

After the adsorption step, liquid feed is switched to the pure iso-octane and effluent is also analyzed to gather the time course data of the DMN concentration in the effluent.

The results of the breakthrough curve and desorption curve are summarized in Fig. 10. As shown in the figure, 2,7-DMN is priorly adsorbed in zeolite compared to 2,6-DMN and it is obviously possible to improve 2,6-/2,7-DMN by contacting DMN isomers with the K-Y type zeolite.

Example-13

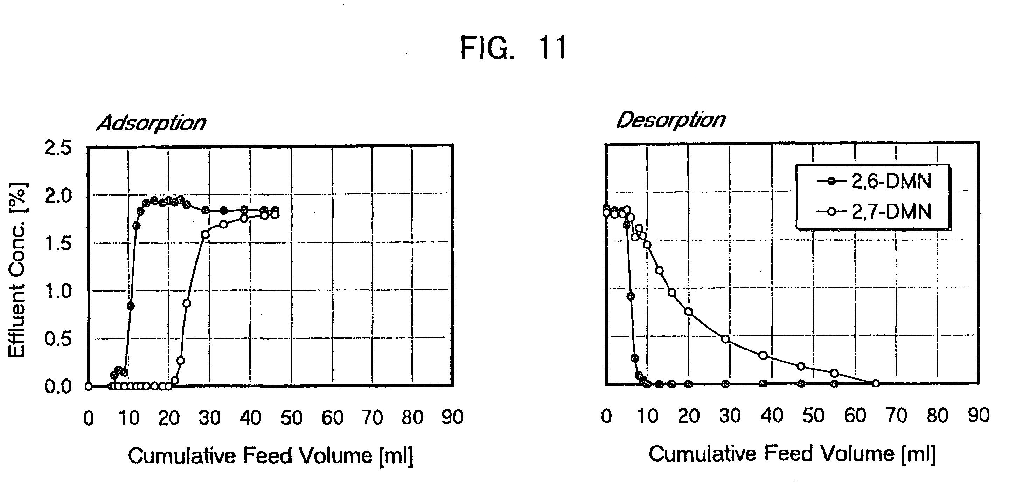

Another adsorption test is carried out in the same manner described in Example 12 except that mesitylene is used as a solvent. The results of the breakthrough curve and desorption curve are summarized in Figure 11.

Obviously, numerous modifications and variations of the present invention are possible in light of the above teachings. It is therefore to be understood that within the scope of the appended claims, the invention may be practiced otherwise than as specifically described herein.

**Claims**

1.  A process for producing 2,6-dialkylnaphthalene from a hydrocarbon feedstock comprising at least one component selected from the group consisting of dialkylnaphthalene isomers, monoalkylnaphthalene isomers, polyalkylnaphthalenes, and naphthalene, comprising the following steps:

    I. separating a dealkylation product fed from step III and, optionally, the hydrocarbon feedstock into a fraction comprising naphthalene, a fraction comprising monoalkyl naphthalene, a fraction comprising dialkylnaphthalene and a fraction comprising remaining products;
    II. separating and purifying 2,6-dialkylnaphthalene from the dialkylnaphthalene fraction of step I;
    III. dealkylating the hydrocarbon feedstock and the remaining products fraction of step I and feeding the dealkylation product to step I;
    IV. alkylating the naphthalene and monoalkylnaphthalene fractions of step I;

    wherein the hydrocarbon feedstock is fed to step III and, optionally, step I.

2.  The process of Claim 1, further comprising dealkylating the dialkylnaphthalene fraction after 2,6-dialkylnaphthalene is separated therefrom in step II and recycling the dealkylation product obtained therefrom to step I.

3.  The process of Claim 1 or 2, wherein dealkylating the dialkylnaphthalene fraction after 2,6-dialkylnaphthalene is separated therefrom in step II is conducted in step III together with dealkylating the hydrocarbon feedstock and the remaining products fraction of step I.

4.  The process of claim 1, wherein the product of step IV is fed to step I.

5.  The process of claim 1, wherein step II comprises the following sub-steps: II-1. separating the dialkylnaphthalene fraction of step I into a 2,6-rich-dialkylnaphthalene fraction and a 2,6-lean-dialkylnaphthalene fraction; II-2, purifying 2,6-dialkylnaphthalene from the 2,5-rich-dialkylnaphthalene fraction from step II-1.

6.  The process of claim 5, further comprising isomerizing at least a part of the 2,6-lean-dialkylnaphthalene fraction from step II-1, wherein at least a part of the isomerization product is fed to step I and/or step II-2.

7.  The process of claim 6, wherein the isomerizing is conducted in the presence of a catalyst composition comprising a synthetic zeolite **characterized by** an X-ray diffraction pattern including interplanar d-spacing (A)
    12.36±0.4
    11.03±0.2
    8.83±0.14
    6.18±0.12
    6.00±0.10
    4.06±0.07
    3.91±0.07
    3.42±0.06.

8. The process of claim 6, wherein at least a part of the product remaining after the 2,6-dialkylnaphthalene is purified in step II-2 is fed to step III.

9. The process of claim 6, wherein at least a part of the product from step IV is fed to step I, and wherein at least a part of the product remaining after the 2,6-dialkylnaphthalene is purified in step II-2 is fed to step III.

10. The process of any preceding claim, wherein the hydrocarbon feedstock is product of pre-processing raw material.

11. The process of claim 10, wherein the pre-processing comprises at least one treatment selected from the group consisting of distillation, concentration, hydrotreatring, de-sulfurization, de-nitrogenation and de-watering.

12. The process of claim 11, wherein the hydrotreating comprises contacting said raw material with a catalyst composition comprising an activated alumina catalyst support comprising an oxide of a Group VIII metal and a Group VI-A metal.

13. The process of claim 12, wherein the Group VIII metal is nickel.

14. The process of claim 12, wherein the Group VI-A metal is molybdenum.

15. The process of claim 12, wherein the oxide of metal is pre-treated at 315.6 - 648.9 °C (600-1200°F) in the presence of a sulfur compound.

16. The process of any of claims 1 to 15, wherein the dealkylation in step I is hydrodealkylation.

17. The process of claim 16, wherein the hydrodealkylation comprises contacting hydrocarbon feedstock with a catalyst composition comprising an activated alumina catalyst support comprising an oxide of a Group VI-A metal.

18. The process of claim 17, wherein the Group VI-A metal is chromium.

19. The process of claim 16, wherein the hydrodealkylation comprises contacting the hydrocarbon feedstock with a catalyst composition comprising an activated alumina catalyst support comprising an oxide of a Group VIII metal, and a Group VI-A metal.

20. The process of claim 19, wherein the Group VIII metal is cobalt.

21. The process of claim 19, wherein the Group VI A metal is molybdenum.

22. The process of claim 19, wherein the oxide of metal is pre-treated at 315.6 - 648.9 °C (600-1000°F) in the presence of organic sulfide.

23. The process of claim 16, wherein the hydrodealkylation is conducted in the presence of a catalyst composition comprising at least a metal selected from the group consisting of noble metal, nickel, combination thereof, and a synthetic zeolite **characterized by** a X-ray diffraction pattern including inter planar d-spacing (A)

      12.36±0.4
      11.03±0.2
      8.83±0.14
      6.18±0.12
      6.00±0.10
      4.06±0.07
      3.91±0.07
      3.42±0.06.

24. The process of any preceding claim, wherein the alkylating is conducted in the presence of a catalyst composition comprising a synthetic zeolite **characterized, by** an X-ray diffraction pattern including inter planar d-spacing (A)

      12.36±0.4
      11.03±0.2
      8.83±0.14
      6.18±0.12

6.00±0.10
4.06±4-07
3.91±0.07
3.42±0.06.

25. The process of any preceding claim, wherein the alkylating agent for the alkylating is methanol or dimethylether.

26. The process of any of claims 5 to 9, wherein the purifying comprises at least one means selected from the group consisting of crystallization under high pressure, cooling crystallization, and fixed bed adsorptive separation.

27. The process of claim 26, wherein the fixed bed adsorptive separation comprises an adsorbent of a zeolite Y containing alkali metal and a desorbent of an organic solvent comprising at least a component selected from the group consisting of hexane, octane, alkylbenzene, and cyclohexane.

28. A process for preparing high-performance polyester resins, comprising

(a) producing 2,6-dialkylnaphthalene according to any of the preceding claims,
(b) oxidising the 2,6-dialkylnaphthalene to 2,6-naphthalenedicarboxylic acid, and
(c) manufacturing the polyester resin from the 2,6-naphthalenedicarboxylic acid.

**Patentansprüche**

1. Verfahren zur Herstellung von 2,6-Dialkylnaphthalin aus einem Kohlenwasserstoff-Ausgangsmaterial, umfassend mindestens eine Komponente, ausgewählt aus der Gruppe, bestehend aus Dialkylnaphthalinisomeren, Monoalkylnaphthalinisomeren, Polyalkylnaphthalinen und Naphthalin, umfassend die folgenden Schritte:

I. das Abtrennen eines Dealkylierungsprodukts, beschickt von Schritt 111, und gegebenenfalls des Kohlenwasserstoff-Ausgangsmaterials in eine Naphthalin umfassende Fraktion, eine Monoalkylnaphthalin umfassende Fraktion, eine Dialkylnaphthalin umfassende Fraktion und eine Fraktion, umfassend verbleibende Produkte;
II. das Abtrennen und Reinigen von 2,6-Dialkylnaphthalin aus der Dialkylnaphthalinfraktion von Schritt I;
III. das Dealkylieren des Kohlenwasserstoff-Ausgangsmaterials und der verbleibenden Produktfraktion von Schritt I und das Beschicken des Dealkylierungsprodukts zu Schritt I;
IV. das Alkylieren der Naphthalin- und Monoalkylnaphthalinfraktionen von Schritt I, wobei die Kohlenwasserstoff-Ausgangsmaterial zu Schritt III und gegebenenfalls Schritt I beschickt wird.

2. Verfahren nach Anspruch 1, weiter umfassend das Dealkylieren der Dialkylnaphthalinfraktion, nachdem 2,6-Dialkylnaphthalin davon in Schritt II abgetrennt worden ist, und das Rückführen des daraus erhaltenen Dealkylierungsproduktes zu Schritt I.

3. Verfahren nach Anspruch 1 oder 2, wobei das Dealkylieren der Dialkylnaphthalinfraktion, nachdem 2,6-Dialkylnaphthalin davon in Schritt II abgetrennt worden ist, in Schritt III zusammen mit dem Dealkylieren des Kohlenwasserstoff-Ausgangsmaterials und der verbleibenden Produktfraktion von Schritt I durchgeführt wird.

4. Verfahren nach Anspruch 1, wobei das Produkt von Schritt IV zu Schritt I beschickt wird.

5. Verfahren nach Anspruch 1, wobei der Schritt II die folgenden Unterschritte umfaßt:

II-1. das Abtrennen der Dialkylnaphthalinfraktion von Schritt I in eine 2,6-reiche Dialkylnaphthalinfraktion und eine 2,6-arme Dialkylnaphthalinfraktion;

II-2. das Reinigen des 2,6-Dialkylnaphthalins von der 2,6-reichen Dialkylnaphthalinfraktion von Schritt II-1.

6. Verfahren nach Anspruch 5, weiter umfassend das Isomerisieren von mindestens einem Teil der 2,6-armen Dialkylnaphthalinfraktion von Schritt II-1, wobei mindestens ein Teil des Isomerisierungsproduktes zu Schritt I und/oder Schritt 11-2 beschickt wird.

7. Verfahren nach Anspruch 6, wobei das Isomerisieren in der Gegenwart einer Katalysatorzusammensetzung durchgeführt wird, umfassend einen synthetischen Zeolithen, **gekennzeichnet durch** ein Röntgenbeugungsmuster einschließlich einem interplanaren d-Abstand (Å):

   12,36 ± 0,4
   11,03 ± 0,2
   8,83 ± 0,14
   6,18 ± 0,12
   6,00 ± 0,10
   4,06 ± 0,07
   3,91 ± 0,07
   3,42 ± 0,06.

8. Verfahren nach Anspruch 6, wobei mindestens ein Teil des Produktes, welches verbleibt, nachdem das 2,6-Dialkylnaphthalin in Schritt II-2 gereinigt worden ist, zu Schritt III beschickt wird.

9. Verfahren nach Anspruch 6, wobei mindestens ein Teil des Produktes von Schritt IV zu Schritt I beschickt wird, und wobei mindestens ein Teil des Produktes, welches verbleibt, nachdem das 2,6-Dialkylnaphthalin in Schritt II-2 gereinigt worden ist, zu Schritt III beschickt wird.

10. Verfahren nach einem vorhergehenden Anspruch, wobei das Kohlenwasserstoff-Ausgangsmaterial ein Produkt eines Vorverarbeitungs-Ausgangsmaterials ist.

11. Verfahren nach Anspruch 10, wobei das Vorverarbeiten mindestens eine Behandlung, ausgewählt aus der Gruppe, bestehend aus Destillation, Konzentration, Hydrotreating, Entschwefelung, Denitrogenierung und Entwässerung, umfaßt.

12. Verfahren nach Anspruch 11, wobei das Hydrotreating das Inkontaktbringen des Ausgangsmaterials mit einer Katalysatorzusammensetzung umfaßt, welche einen aktivierten Aluminiumoxidkatalysatorträger, umfassend ein Oxid eines Metalls der Gruppe VIII und eines Metalls der Gruppe VI-A, umfaßt.

13. Verfahren nach Anspruch 12, wobei das Metall der Gruppe VIII Nickel ist.

14. Verfahren nach Anspruch 12, wobei das Metall der Gruppe VI-A Molybdän ist.

15. Verfahren nach Anspruch 12, wobei das Metalloxid bei 315,6-648,9°C (600-1200°F) in der Gegenwart einer Schwefelverbindung vorbehandelt worden ist.

16. Verfahren nach einem der Ansprüche 1 bis 15, wobei die Dealkylierung in Schritt I eine Hydrodealkylierung ist.

17. Verfahren nach Anspruch 16, wobei die Hydrodealkylierung das Inkontaktbringen des Kohlenwasserstoff-Ausgangsmaterials mit einer Katalysatorzusammensetzung umfaßt, welche einen aktivierten Aluminiumoxidkatalysatorträger, umfassend ein Oxid eines Metalls der Gruppe VI-A, umfaßt.

18. Verfahren nach Anspruch 17, wobei das Metall der Gruppe VI-A Chrom ist.

19. Verfahren nach Anspruch 16, wobei die Hydrodealkylierung das Inkontaktbringen des Kohlenwasserstoff-Ausgangsmaterials mit einer Katalysatorzusammensetzung umfaßt, welche einen aktivierten Aluminiumoxidkatalysatorträger, umfassend ein Oxid eines Metalls der Gruppe VIII und eines Metalls der Gruppe VI-A, umfaßt.

20. Verfahren nach Anspruch 19, wobei das Metall der Gruppe VIII Kobalt ist.

21. Verfahren nach Anspruch 19, wobei das Metall der Gruppe VI-A Molybdän ist.

22. Verfahren nach Anspruch 19, wobei das Metalloxid bei 315,6-648,9°C (600-1000°F) in der Gegenwart eines organischen Sulfids vorbehandelt worden ist.

23. Verfahren nach Anspruch 16, wobei die Hydrodealkylierung in der Gegenwart einer Katalysatorzusammensetzung durchgeführt wird, welche mindestens ein Metall, ausgewählt aus der Gruppe, bestehend aus Edelmetall, Nickel und einer Kombination davon, und einen synthetischen Zeolithe, **gekennzeichnet durch** ein Röntgenbeugungsmuster einschließlich einem interplanaren d-Abstand (Å)

12,36 ± 0,4
11,03 ± 0,2
8,83 ± 0,14
6,18 ± 0,12
6,00 ± 0,10
4,06 ± 0,07
3,91 ± 0,07
3,42 ± 0,06,

umfasst.

24. Verfahren nach einem vorhergehenden Anspruch, wobei die Alkylierung in der Gegenwart einer Katalysatorzusammensetzung durchgeführt wird, umfassend einen synthetischen Zeolith, **gekennzeichnet durch** ein Röntgenbeugungsmuster einschließlich einem interplanaren d-Abstand (Å)

12,36 ± 0,4
11,03 ± 0,2
8,83 ± 0,14
6,18 ± 0,12
6,00 ± 0,10
4,06 ± 0,07
3,91 ± 0,07
3,42 ± 0,06.

25. Verfahren nach einem vorhergehenden Anspruch, wobei das Alkylierungsmittel für die Alkylierung Methanol oder
Dimethylether ist.

26. Verfahren nach einem der Ansprüche 5 bis 9, wobei das Reinigen mindestens ein Mittel umfaßt, ausgewählt aus
der Gruppe, bestehend aus Kristallisation unter hohem Druck, Kühlkristallisation und adsorptive Trennung im Festbett.

27. Verfahren nach Anspruch 26, wobei die adsorptive Trennung im Festbett ein Adsorptionsmittel eines Zeolithen Y,
enthaltend Alkalimetall, und ein Desorptionsmittel eines organischen Lösungsmittels, umfassend mindestens eine
Komponente, ausgewählt aus der Gruppe, bestehend aus Hexan, Octan, Alkylbenzol und Cyclohexan, umfaßt.

28. Verfahren zur Herstellung von High-Performance-Polyesterharzen, umfassend

(a) das Herstellen von 2,6-Dialkylnaphthalin gemäß einem der vorhergehenden Ansprüche,
(b) das Oxidieren des 2,6-Dialkylnaphthalins zu 2,6-Naphthalindicarbonsäure, und
(c) das Herstellen des Polyesterharzes aus der 2,6-Naphthalindicarbonsäure.

**Revendications**

1. Procédé de production de 2,6-dialkylnaphtalène à partir d'une charge hydrocarbonée comprenant au moins un
composant choisi dans le groupe constitué par les isomères de dialkylnaphtalène, les isomères de monoalkylnaphtalène, les polyalkylnaphtalènes et le naphtalène, comprenant les étapes suivantes consistant à :

I. séparer un produit de désalkylation provenant de l'étape III et, éventuellement, la charge hydrocarbonée en
une fraction comprenant le naphtalène, une fraction comprenant le monoalkylnaphtalène, une fraction comprenant le dialkylnaphtalène et une fraction comprenant les produits restants ;
II. séparer et purifier le 2,6-dialkylnaphtalène de la fraction dialkylnaphtalène de l'étape I ;
III. désalkyler la charge hydrocarbonée et la fraction de produits restants de l'étape I et charger le produit de
désalkylation dans l'étape I ;
IV. alkyler les fractions naphtalène et monoalkylnaphtalène de l'étape I ;

dans lequel la charge hydrocarbonée est chargée dans l'étape III et, éventuellement, dans l'étape I.

**2.** Procédé selon la revendication 1, comprenant en outre la désalkyltion de la fraction dialkylnaphtalène après en avoir séparé le 2,6-dialkylnaphtalène dans l'étape II et le recyclage du produit de désalkylation obtenu de celle-ci dans l'étape I.

**3.** Procédé selon la revendication 1 ou 2, dans lequel la désalkylation de la fraction dialkylnaphtalène après en avoir séparé le 2,6-dialkylnaphtalène dans l'étape II est réalisée dans l'étape III avec la désalkylation de la charge hydrocarbonée et de la fraction de produits restants de l'étape I.

**4.** Procédé selon la revendication 1, dans lequel le produit de l'étape IV est chargé dans l'étape I.

**5.** Procédé selon la revendication 1, dans lequel l'étape II comprend les sous-étapes suivantes :

II-1 la séparation de la fraction dialkylnaphtalène de l'étape I en une fraction riche en 2,6-dialkylnaphtalène et une fraction pauvre en 2,6-dialkylnaphtalène ;
II.2 la purification du 2,6-dialkylnaphtalène provenant de la fraction riche en 2,6-dialkylnaphtalène de l'étape II.1.

**6.** Procédé selon la revendication 5, comprenant en outre l'isomérisation d'au moins une partie de la fraction pauvre en 2,6-dialkylnaphtalène de l'étape II.1, dans lequel au moins une partie du produit d'isomérisation est chargée dans l'étape I et/ou dans l'étape II.2.

**7.** Procédé selon la revendication 6, dans lequel l'isomérisation est réalisée en présence d'une composition de catalyseur comprenant une zéolite synthétique **caractérisée par** un spectre de diffraction des rayons X comprenant l'écartement interplanaire d (Å)

$12,36 \pm 0,4$
$11,03 \pm 0,2$
$8,83 \pm 0,14$
$6,18 \pm 0,12$
$6,00 \pm 0,10$
$4,06 \pm 0,07$
$3,91 \pm 0,07$
$3,42 \pm 0,06.$

**8.** Procédé selon la revendication 6, dans lequel au moins une partie du produit restant après la purification du 2,6-dialkylnaphtalène dans l'étape II.2 est chargée dans l'étape III.

**9.** Procédé selon la revendication 6, dans lequel au moins une partie du produit provenant de l'étape IV est chargée dans l'étape I, et dans lequel au moins une partie du produit restant après la purification du 2,6-dialkylnaphtalène dans l'étape II.2 est chargée dans l'étape III.

**10.** Procédé selon l'une quelconque des revendications précédentes, dans lequel la charge hydrocarbonée est un produit d'un pré-traitement d'une matière première.

**11.** Procédé selon la revendication 10, dans lequel le pré-traitement comprend au moins un traitement choisi dans le groupe constitué par une distillation, une concentration, un hydrotraitement, une désulfuration, une désazotisation et un égouttage.

**12.** Procédé selon la revendication 11, dans lequel l'hydrotraitement comprend la mise en contact de ladite matière première avec une composition de catalyseur comprenant un support de catalyseur de type alumine activée comprenant un oxyde d'un métal du groupe VIII et d'un métal du groupe VI-A.

**13.** Procédé selon la revendication 12, dans lequel le métal du groupe VIII est le nickel.

**14.** Procédé selon la revendication 12, dans lequel le métal du groupe VI-A est le molybdène.

**15.** Procédé selon la revendication 12, dans lequel l'oxyde de métal est prétraité à une température de 315,6 à 648,9°C (600 à 1200°F) en présence d'un composé du soufre.

**16.** Procédé selon l'une quelconque des revendications 1 à 15, dans lequel la désalkylation dans l'étape I est une hydrodésalkylation.

**17.** Procédé selon la revendication 16, dans lequel l'hydrodésalkylation comprend la mise en contact de la charge hydrocarbonée avec une composition de catalyseur comprenant un support de catalyseur de type alumine activée comprenant un oxyde d'un métal du groupe VI-A.

**18.** Procédé selon la revendication 17, dans lequel le métal du groupe VI-A est le chrome.

**19.** Procédé selon la revendication 16, dans lequel l'hydrodésalkylation comprend la mise en contact de la charge hydrocarbonée avec une composition de catalyseur comprenant un support de catalyseur de type alumine activée comprenant un oxyde d'un métal du groupe VIII et un métal du groupe VI-A.

**20.** Procédé selon la revendication 19, dans lequel le métal du groupe VIII est le cobalt.

**21.** Procédé selon la revendication 19, dans lequel le métal du groupe VI-A est le molybdène.

**22.** Procédé selon la revendication 19, dans lequel l'oxyde de métal est prétraité à une température de 315,6 à 648,9°C (600 à 1000°F) en présence d'un sulfure organique.

**23.** Procédé selon la revendication 16, dans lequel l'hydrodésalkylation est réalisée en présence d'une composition de catalyseur comprenant au moins un métal choisi dans le groupe constitué par un métal noble, le nickel, une combinaison de ceux-ci et une zéolite synthétique **caractérisée par** un spectre de diffraction des rayons X comprenant l'écartement interplanaire d (Å)

12,36 ± 0,4
11,03 ± 0,2
8,83 ± 0,14
6,18 ± 0,12
6,00 ± 0,10
4,06 ± 0,07
3,91 ± 0,07
3,42 ± 0,06.

**24.** Procédé selon l'une quelconque des revendications précédentes, dans lequel l'alkylation est réalisée en présence d'une composition de catalyseur comprenant une zéolite synthétique **caractérisée par** un spectre de diffraction des rayons X comprenant l'écartement interplanaire d (Å)

12,3 ± 0,4
11,03 ± 0,2
8,83 ± 0,14
6,18 ± 0,12
6,00 ± 0,10
4,06 ± 0,07
3,91 ± 0,07
3,42 ± 0,06.

**25.** Procédé selon l'une quelconque des revendications précédentes, dans lequel l'agent d'alkylation pour l'alkylation est le méthanol ou le diméthyléther.

**26.** Procédé selon l'une quelconque des revendications 5 à 9, dans lequel la purification comprend au moins un moyen choisi dans le groupe constitué par une cristallisation sous pression élevée, une cristallisation par refroidissement et une séparation par adsorption sur un lit fixe.

**27.** Procédé selon la revendication 26, dans lequel la séparation par adsorption sur un lit fixe comprend un adsorbant d'une zéolite Y contenant un métal alcalin et un désorbant d'un solvant organique comprenant au moins un composant choisi dans le groupe constitué par l'hexane, l'octane, un alkylbenzène et le cyclohexane.

**28.** Procédé de préparation de résines de polyester haute performance comprenant :

(a) la production de 2,6-dialkylnaphtalèe selon l'une quelconque des revendications précédentes,
(b) l'oxydation du 2,6-dialkylnaphtalène en acide 2,6-naphtalènedicarboxylique, et
(c) la fabrication de la résine de polyester à partir de l'acide 2,6-naphtalènedicarboxylique.

# F I G. 1

# F I G. 2

FEEDSTOCK NL, MAN, DAN, PAN (OPTIONAL)

FEEDSTOCK NL, MAN, DAN, PAN

LIGHTS

MAN RICH STREAM

ALKYLATING AGENT

NL RICH STREAM

(I) SEPARATION

(III) DEALKYLATION

PAN RICH STREAM

DAN RICH STREAM

(IV) ALKYLATION → MAN, DAN, PAN, NL

DEALKYLATION

REMAINING PRODUCTS (2,6-LEAN-DAN)

(II) SEPARATION / PURIFICATION → 2,6-DAN

# F I G. 3

ALKYLATING AGENT

(IV) ALKYLATION → MAN, DAN, PAN, NL

MAN
RICH STREAM

NL
RICH STREAM

LIGHTS

REMAINING
PRODUCTS
(2,6-LEAN-DAN)

FEEDSTOCK
NL, MAN,
DAN, PAN
(OPTIONAL)

(I) SEPARATION

DAN
RICH STREAM

(II) SEPARATION
/PURIFICATION → 2,6-DAN

PAN RICH STREAM

FEEDSTOCK
NL, MAN,
DAN, PAN

(III) DEALKYLATION

EP 1 165 473 B1

# FIG.4

ALKYLATING AGENT

(IV) ALKYLATION → MAN, DAN, PAN, NL

MAN RICH STREAM

NL RICH STREAM

LIGHTS

REMAINING PRODUCTS (2,6-LEAN-DAN)

FEEDSTOCK NL, MAN, DAN, PAN (OPTIONAL) → (I) SEPARATION

DAN RICH STREAM → (II) SEPARATION / PURIFICATION → 2,6-DAN

PAN RICH STREAM

FEEDSTOCK NL, MAN, DAN, PAN → (III) DEALKYLATION

EP 1 165 473 B1

# F I G. 5

EP 1 165 473 B1

# FIG.6

EP 1 165 473 B1

# FIG. 7

EP 1 165 473 B1

# FIG.8

# FIG. 9
## (CRYSTALLIZATION UNDER HIGH PRESSURE)

# FIG. 10

*Adsorption*

Effluent Conc. [%]

Cumulative Feed Volume [ml]

*Desorption*

- 2,6-DMN
- 2,7-DMN

Cumulative Feed Volume [ml]

EP 1 165 473 B1

FIG. 11